# EUROPÄISCHE PATENTSCHRIFT

(11) **EP 0 874 607 B1**
(45) Veröffentlichungstag und Bekanntmachung des Hinweises auf die Patenterteilung: **26.11.2003**
(21) Anmeldenummer: 96942221.1
(22) Anmeldetag: 19.12.1996
(51) Int. Cl.: A61F 5/058

(54) **MEDIZINISCHE SCHIENE SOWIE VERFAHREN ZUM HERSTELLEN EINER SOLCHEN SCHIENE**
MEDICAL SPLINT AND METHOD OF ITS MANUFACTURING
ATTELLE MEDICALE ET METHODE DE FABRICATION DE CETTE ATTELLE

(30) Priorität: 20.12.1995 CH 360595; 08.08.1996 CH 195096
(43) Veröffentlichungstag der Anmeldung: 04.11.1998
(73) Patentinhaber: Chrisofix AG, 8201 Schaffhausen (CH)
(72) Erfinder: BOLLA, Kalman, CH-8212 Neuhausen am Rheinfall (CH); BOLLA, Orsolya, CH-8212 Neuhausen am Rheinfall (CH)
(74) Vertreter: Isler & Pedrazzini AG
(86) Internationale Anmeldenummer: CH9600450
(87) Internationale Veröffentlichungsnummer: WO97022312

(56) Entgegenhaltungen:
- BE-A- 666 752
- US-A- 2 273 028
- US-A- 2 667 868
- US-A- 3 850 167
- US-A- 4 161 175

## Beschreibung

### TECHNISCHES GEBIET

Die vorliegende Erfindung bezieht sich auf das Gebiet der medizinischen Schienen für die Human- aber auch für die Tiermedizin. Sie betrifft eine medizinische Schiene zum Fixieren bzw. Ruhigstellen von beweglichen Körperteilen gemäss dem Oberbegriff des Anspruchs 1 sowie ein Verfahren zu deren Herstellung.

Metallschienen sind nicht ungewöhnlich. Eine solche Schiene ist z.B. aus der Druckschrift US-A-3,943,923 bekannt.

### STAND DER TECHNIK

Schienen, die im wesentlichen aus einem leicht plastisch verformbaren Metallblech (meist aus Al) bestehen, welches einoder beidseitig mit einer Abdeckschicht aus einem Kunststoffschaum oder dgl. bedeckt ist, sind aus dem Stand der Technik in vielen Varianten bekannt. Die Schienen liegen meist als ebene Platten vor, die im Anwendungsfall durch plastisches Verbiegen in einem allerdings eingeschränkten Umfang an die zu schienenden Gliedmassen angepasst werden, um durch Anliegen den notwendigen Halt zu geben bzw. die erforderliche Fixierung zu ermöglichen. Das innenliegende Metallblech kann dabei entweder praktisch die gesamte Fläche der Schiene ausfüllen, wie dies in der US-A-3,943,923, der US-A-4,676,233 oder der EP-B1-0 039 323 der Fall ist, oder es wird in Form mehrerer, versteifender Metallstreifen in die Schiene eingebettet, wie dies bei der EP-B1-0 073 772 der Fall ist.

Problematisch ist die gleichzeitige Erfüllung einander widerstrebender Forderungen bei solchen anpassbaren oder anformbaren Schienen: Zum einen soll die Schiene genügend leicht plastisch verformbar bzw. verbiegbar sein, damit sie von Hand ohne zusätzliche Hilfsmittel an die zu fixierenden Gliedmassen (Körperteile) angepasst werden kann. Zum anderen soll die Schiene aber trotz der leichten Verformbarkeit hinreichend steif sein, um die erforderliche Fixierung der Gliedmassen zu gewährleisten.

Um die nötige Stabilität der Schiene zu erreichen, ist z.B. in der US-A-4,161,175 vorgeschlagen worden, zur Versteifung des Metallblechs in Richtung der Schienenachse verlaufende gerade oder sinusartig hin- und herschwingende Rippen in das Blech einzuformen. Eine ähnliche Versteifung wird in der US-A-4,676,233 durch das nachträgliche Biegen einer in Schienenachse verlaufenden Rippe erzeugt.

Aus der US-A-3,850,167 ist eine Schiene bekannt, bei der zwei Wellpappen mit ihren Wellen orthogonal zueinander orientiert miteinander in einer vorgegebenen U-Form miteinander verklebt werden, um eine mechanisch steife Schiene zu erzeugen.

Ein noch grösseres Problem besteht darin, dass sich ein glattes Metallblech wegen der bei normalen Kräften nur geringen Dehn- bzw. Stauchbarkeit in der Blechebene nur sehr schlecht durch plastisches Verbiegen an die unregelmässigen und teilweise auch stark gekrümmten Konturen der zu fixierenden bzw. ruhigzustellenden Gliedmassen oder Körperteile formgerecht anpassen lässt. Besonders schwierig wird eine solche Anpassung, wenn die Schiene eine grössere, einfach zusammenhängende Fläche aufweist. Dieses Problem wird von den bisher bekannten Schienen mit Metallblech nicht gelöst. Eine gute Anpassung an kompliziert geformte Gliedmassen und Körperteile ist bislang vielmehr nur durch herkömmliche Gipsschienen oder thermisch verformbare Kunststoffschienen erreichbar.

### DARSTELLUNG DER ERFINDUNG

Es ist daher Aufgabe der Erfindung, eine medizinische Schiene zu schaffen, die von der Form einer ebenen Platte ausgehend mit geringem Kraftaufwand von Hand leicht an die zu fixierenden Gliedmassen bzw. Körperteile angepasst werden kann, im angeformten Zustand die notwendige Steifigkeit aufweist, und flexibel in der Anwendung ist, sowie ein Verfahren zu deren Herstellung anzugeben.

Die Aufgabe wird durch die Gesamtheit der Merkmale der Ansprüche 1, 23 und 24 gelöst.

Der Kern der Erfindung besteht darin, durch die Wellung des Metallblechs eine leichte Dehnbarkeit und Stauchbarkeit in der Schienenebene zu erzeugen, so dass sich die Schiene ohne grosse Schwierigkeiten lokalen Unebenheiten an den zu fixierenden Gliedmassen oder Körperteilen anpassen lässt. Ist eine lokale Dehnung erforderlich, ziehen sich die Wellen im Metallblech in diesem Bereich auseinander. Ist dagegen eine lo kale Stauchung notwendig, werfen sich die Wellen in diesem Bereich steiler auf und rücken enger zusammen. Auf diese Weise erhält die Schiene eine aussergewöhnlich hohe lokale Verformbarkeit. Ein weiteres wichtiges Merkmal der erfindungsgemässen Schiene ist die Orientierung der Wellen quer zur Schienenachse. Wenn die Schiene mit der Schienenachse parallel zu den zu fixierenden Gliedmassen (Körperteilen) angelegt wird und die Schiene zur Anpassung an die Gliedmassen quer zur Schienenachse z.B. U-förmig um die Gliedmassen herum gebogen wird, ergibt sich durch die spezielle erfindungsgemässe Orientierung der Wellen eine überraschend starke Versteifung der Schiene nach dem Umbiegen, so dass mit dem gewellten Metallblech gleichzeitig eine hohe Anformbarkeit und gute Fixierung (Schienung) erreicht wird.

Besonders günstige Verformungs- und Fixierungseigenschaften hat die Schiene dadurch, dass das Metallblech aus Aluminium besteht, das Metallblech eine Dicke von kleiner gleich 1 mm, vorzugsweise kleiner gleich 0,3 mm, aufweist, die Wellen untereinander einen periodischen Abstand von zwischen 1 mm und 8 mm, insbesondere etwa 3-5 mm, aufweisen, und die Wellen eine Höhe zwischen den Wellenbergen und Wellentälern von zwischen 0,5 mm und 5 mm, insbesondere zwischen 1 mm und 3 mm, aufweisen.

Eine erste bevorzugte Ausführungsform der erfindungsgemässen Schiene ist dadurch gekennzeichnet, dass das Metallblech über die gesamte Fläche gewellt ausgebildet ist, und die Wellen quer zur Schienenachse zwischen gegenüberliegenden Rändern des Metallbleches durchgehend ausgebildet sind. Hierdurch lässt sich die Schiene flexibel für die unterschiedlichsten Schienungen einsetzen.

Zur Abpolsterung und Erhöhung des Tragekomforts hat es sich als besonders günstig erwiesen, wenn bei der Schiene gemäss einer weiteren bevorzugten Ausführungsform die Abdeckung Abdeckschichten umfasst, welche aus einem Kunststoff, insbesondere aus einem Schaumstoff, vorzugsweise aus einem elastischen Polyäthylen- oder Polyurethanschaum, bestehen, der beispielsweise eine Schichtdicke von 1 bis 3 mm aufweist. Selbstverständlich kann in bestimmten Anwendungsfällen die Polsterung auch wesentlich dicker (z.B. im cm-Bereich) sein. Um den Tragekomfort weiter zu erhöhen, können eine der Abdeckschichten oder beide Abdeckschichten auf der Aussenseite zusätzlich mit einer Schicht aus Textil laminiert sein.

Das Metallblech lässt sich einfach und sicher zwischen den Abdeckschichten einbetten, wenn gemäss einer weiteren bevorzugten Ausführungsform die Abdeckschichten über den Rand des Metallbleches hinausragen und einen umlaufenden Randbereich bilden, in welchem die beiden Abdeckschichten miteinander verbunden, vorzugsweise verklebt oder verschweisst, sind.

Für allgemeine Anwendungen als Unterarmschiene oder dgl. ist es günstig, wenn die Schiene gemäss einer anderen Ausführungsform eine im wesentlichen rechteckige Randkontur aufweist.

Für spezielle Anwendungen, z.B. als Fingerschiene (Metacarpalschiene), Daumenschiene oder Unterarmstütze, ist es günstig, wenn die Schiene gemäss einer weiteren Ausführungsform in ihrer Randkontur dem jeweiligen Einsatzbereich angepasst ist.

Eine weitere bevorzugte Ausführungsform der erfindungsgemässen Schiene zeichnet sich dadurch aus, dass an einer Stelle oder mehreren Stellen der Schiene Mittel zum lösbaren oder dauerhaften Verbinden der Schiene mit zusätzlichen Befestigungsmitteln vorgesehen sind. Hierdurch kann die Schiene nach der Anpassung an die Gliedmassen einfach und flexibel mittels zusätzlicher Befestigungsmittel wie z.B. Riemen zusätzlich fixiert und gesichert werden.

Besonders bewährt hat sich in diesem Zusammenhang, wenn die Verbindungsmittel einen in der Ebene der Schiene angebrachten Druckknopf bzw. Druckknopfteil umfassen.

Bevorzugt kann die erfindungsgemässe Schiene beim Menschen als Unterarm- bzw. Handgelenkschiene/stütze, als Daumenschiene, als Fingerschiene, oder als Bein- und/oder Fussschiene eingesetzt werden.

Eine bevorzugte Ausführungsform der erfindungsgemässen Schiene in Form einer Fingerschiene für die Fixierung eines einzelnen verletzten Fingers oder mehrerer verletzter Finger, ist dadurch gekennzeichnet, dass das Metallblech eine längliche, in der Längsrichtung durch zwei Längskanten berandete Platte ist, dass die Platte von den Längskanten berandete Seitenabschnitte aufweist, welche aus der Plattenebene heraus gebogen werden können, um als Seitenwände dem in der Fingerschiene gelagerten Finger seitlich Halt zu geben und gleichzeitig einen Beitrag zur Längssteifigkeit der Fingerschiene zu leisten, und dass in den Seitenabschnitten eine Mehrzahl von in die Platte eingebrachten Wellen vorgesehen sind, welche sich von den Längskanten aus quer in die Seitenabschnitte bzw. Seitenwände hinein erstrecken.

Weitere Ausführungsformen ergeben sich aus den abhängigen Ansprüchen.

### KURZE ERLÄUTERUNG DER FIGUREN

Die Erfindung soll nachfolgend anhand von Ausführungsbeispielen im Zusammenhang mit der Zeichnung näher erläutert werden.
Es zeigen
- Fig. 1: ein bevorzugtes Ausführungsbeispiel einer erfindungsgemässen Schiene in allgemein anwendbarer rechteckiger Form in der perspektivischen Ansicht (A) und im Längsschnitt (B);
- Fig. 2: ein Ausführungsbeispiel für eine als spezielle Fingerschiene (Metacarpalschiene) ausgebildete Schiene nach der Erfindung (A) und ein als Riemen ausgebildetes zusätzliches Befestigungsmittel (B) für die Fingerschiene (oder auch andere Schienenformen);
- Fig. 3: ein mit spezieller Randkontur versehenes Ausführungsbeispiel der erfindungsgemässen Schiene für eine Anwendung als Daumenschiene mit Handgelenkfixierung;
- Fig. 4,4A: die Anwendung einer Schiene nach der Erfindung als Unterarm- bzw. Handgelenkstütze oder - schiene.
- Fig. 5: in einer Seitenansicht ein Ausführungsbeispiel einer (wannenförmig gebogenen) Schiene nach der Erfindung als Fingerschiene mit fortlaufenden Wellen;
- Fig. 6: in der Draufsicht (A) und im Längsschnitt (B) eine unverformte Platte für eine Fingerschiene der in Fig. 5 dargestellten Art;
- Fig. 7: in der Draufsicht eine Fingerschiene gemäss Fig. 6 als (integraler) Teil einer Armschiene; und
- Fig. 8: in schematischer Darstellung ein Verfahren zur Herstellung der gewellten Platte einer Fingerschiene gemäss Fig. 5 bis 7.

### WEGE ZUR AUSFÜHRUNG DER ERFINDUNG

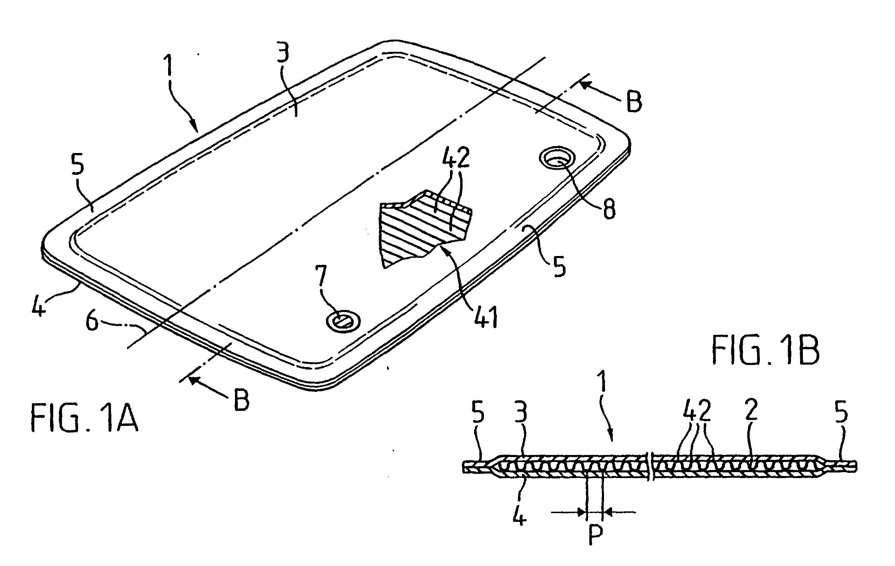

In Fig. 1 ist ein bevorzugtes Ausführungsbeispiel einer erfindungsgemässen Schiene in allgemein anwendbarer rechteckiger Form dargestellt. Fig. 1A zeigt dabei die Schiene in perspektivischer Ansicht. Fig. 1B zeigt den Längsschnitt durch die Schiene entlang der Linie B-B in Fig. 1A. Die Schiene 1 hat in diesem Ausführungsbeispiel eine längliche, rechteckige Form mit vorzugsweise abgerundeten Ecken. Zentraler Bestandteil der Schiene 1 ist ein dünnes Metallblech 2 aus Aluminium, welches durchgehend mit einer feinen Wellung (Wellen 42 im aufgeschnittenen Bereich 41 in Fig. 1A) versehen ist. Die äusseren Abmessungen der Schiene 1 können sehr unterschiedlich sein. Als allgemeine Unterarmschiene eignet sich beispielsweise eine Schiene 1 mit einer Breite von 10 - 20 cm und einer Länge von 20 - 30 cm.

Selbstverständlich sind auch andere Abmessungen denkbar. Weiterhin ist es auch denkbar, dass an der Schiene Formanpassungen in Form von Aussparungen, Einschnitten, Ansätzen oder dgl. vorgesehen sind. Ebenfalls ist es denkbar und vorteilhaft, in bestimmten Bereichen der Schiene mit erhöhter Stützwirkung Verstärkungen z.B. in Form von eingelegten Blechstreifen oder -platten vorzusehen. Auch ist es denkbar, durch bereichsweise Aenderung der Wellenrichtung, insbesondere senkrecht zur vorherrschenden Wellenrichtung, die Schiene bereichsweise zu versteifen. Die Längsachse der Schiene 1 bildet zugleich die Schienenachse 6, die bei der Anwendung im wesentlichen parallel zu den zu fixierenden Gliedmassen, z.B. einem Arm, orientiert ist.

Die Wellung des Metallbleches 2 ist gegenüber den äusseren Abmessungen sehr klein: Der periodische Abstand P der Wellen 42 beträgt zwischen 1 mm und 8 mm und beträgt insbesondere etwa 3-5 mm. Die Wellen 42 weisen eine Höhe zwischen den Wellenbergen und Wellentälern von ebenfalls zwischen 1 mm und 8 mm, insbesondere zwischen 2 mm und 5 mm, auf. Das Metallblech 2 aus Aluminium hat eine Dicke von kleiner gleich 1 mm, vorzugsweise kleiner gleich 0,3 mm. Die Wellung des Metallbleches 2 kann beispielsweise dadurch erzeugt werden, dass ein ebenes Metallblech durch zwei gegenläufig rotierende, ineinandergreifende Zahnradwalzen gezogen oder zwischen zwei entsprechend gewellten Stempeln gepresst wird.

Grundsätzlich könnte das gewellte Metallblech 2 an sich als Schiene eingesetzt werden. Zur Polsterung und thermischen Isolierung (Kältegefühl von Metall auf der Haut) ist das Metallblech 2 innerhalb der Schiene 1 beidseitig mit einer Abdeckschicht 3 bzw. 4 abgedeckt. Als Abdeckschicht kann ein Stoffgewebe, ein Filz oder dgl. verwendet werden. Bevorzugt bestehen die Abdeckschichten 3, 4 jedoch aus einem Kunststoff, insbesondere einem Schaumstoff, vorzugsweise aus einem elastischen Polyäthylen- oder Polyurethanschaum mit einer Dicke von wenigen Millimetern, der flexibel, elastisch und hygienisch unbedenklich ist. Wie aus Fig. 1B zu ersehen ist, ragen die Abdeckschichten 3, 4 über den Rand des Metallbleches 2 hinaus. Sie bilden einen gleichmässigen, umlaufenden Randbereich 5, in welchem die beiden Abdeckschichten 3, 4 miteinander verbunden, vorzugsweise verklebt oder verschweisst, sind. Gleichzeitig sind die Abdeckschichten 3, 4 auf ihrer Innenfläche mit dem Metallblech 2 verbunden (z.B. verschweisst). Die Abdeckschichten 3, 4 bilden so eine geschlossene Umhüllung, welche das gewellte Metallblech 2 aufnimmt und allseitig umgibt.

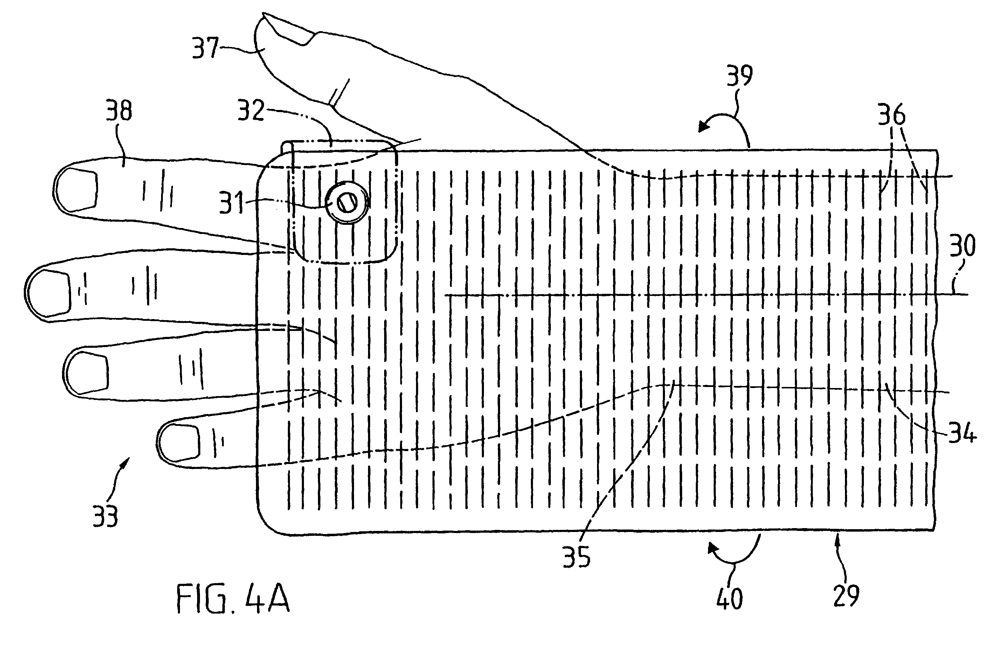

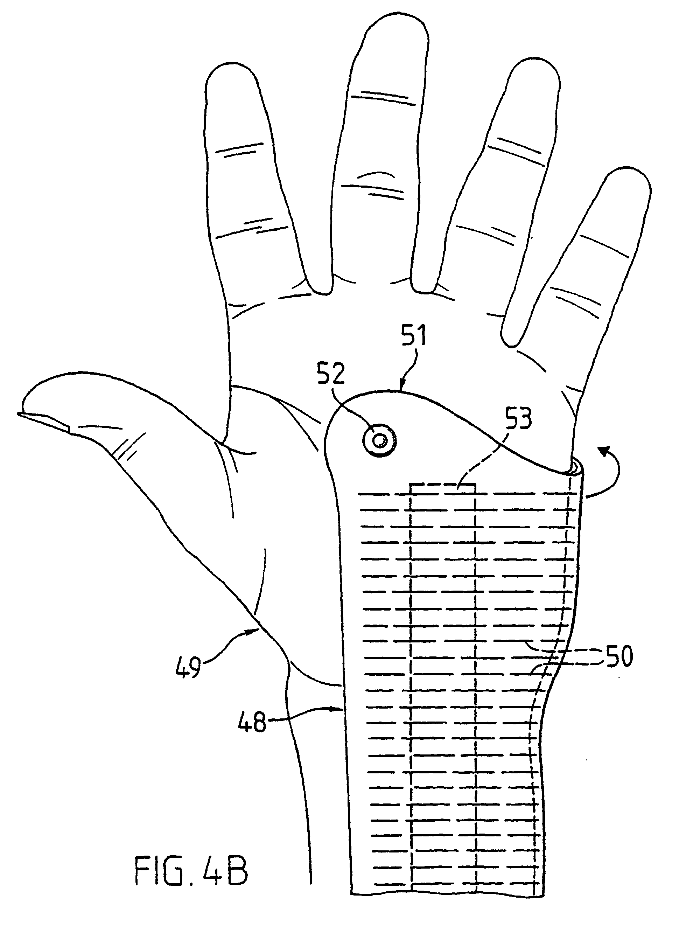

Die Schiene 1 wird bei der Anwendung mit der Schienenachse 6 parallel zu den Gliedmassen (Arm oder dgl.) bzw. Körperteilen (Hals, Rumpf) angelegt und durch Biegen quer zur Schienenachse U-förmig an die Gliedmassen (Körperteile) angepasst und angeformt (siehe auch Fig. 4, 4A). Die Schiene muss dabei nicht unbedingt als ebene Platte vorliegen, sondern kann bereits wannenförmig vorgeformt sein, um die Anwendung zu erleichtern und eine fehlerhafte Orientierung beim Einsatz auszuschliessen. Die Wellung des Metallbleches 2 ermöglicht dabei auf einfache Weise eine lokale Stauchung oder Dehnung in der Ebene des Metallbleches 2, so dass sich die Schiene auch an sehr unregelmässig geformte Gliedmassen (Körperteile) leicht durch Biegen per Hand anpassen lässt.

Zugleich sorgt die spezielle Orientierung der von einem Rand des Metallbleches 2 zum anderen Rand quer zur Schienenachse 6 durchgehenden Wellen 42 überraschenderweise dafür, dass die Schiene 1 nach dem Umbiegen eine ungewöhnlich hohe Steifigkeit aufweist, obgleich sie im ungebogenen Zustand zunächst sehr biegsam ist. Insbesondere kann eine lokale Erhöhung der Steifigkeit dadurch herbeigeführt werden, dass die Wellen in diesem Bereich mit dem Daumen oder auch anderweitig flachgedrückt bzw. aus dem Blech herausgedrückt werden. Trotz der durch das Umbiegen bewirkten Steifigkeit bleibt die Schiene überraschenderweise weiterhin gut modellierbar, so dass sie zu jeder Zeit den Körperteilen bzw. Gliedmassen auch erneut angepasst werden kann.

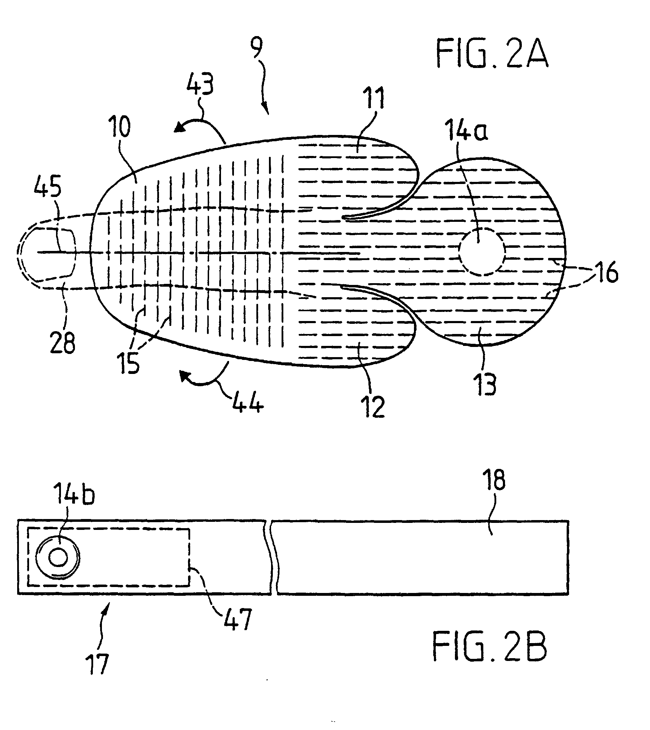

Die Schiene kann im angeformten, gebogenen Zustand mit zusätzlichen Befestigungsmitteln wie z.B. einem Riemen (Fig. 2B) oder dgl. an den geschienten Gliedmassen (Körperteilen) befestigt werden, um ein Abfallen der Schiene sicher auszuschliessen. Um eine lösbare Verbindung derartiger Befestigungsmittel mit der Schiene zu ermöglichen, können an geeigneten Stellen der Schiene 1 (z.B. in Eckbereichen) Verbindungsmittel vorgesehen sein. Als Verbindungsmittel kommen gemäss Fig. 1A beispielsweise Druckknöpfe bzw. Druckknopfteile 7 oder Oesen 8 in Betracht. Es ist aber auch denkbar, andere Verbindungsmittel wie z.B. Klebeflächen oder Klettverbinder oder Haken vorzusehen. Werden Oesen 8 eingesetzt, werden diese in der Schienenebene durch die Schichtenfolge aus unterer Abdeckschicht 4, Metallblech 2 und oberer Abdeckschicht 3 hindurch angebracht. Bevorzugt werden jedoch Druckknöpfe bzw. Druckknopf teile 7, die jeweils nur durch das Metallblech 2 und eine der Abdeckschichten 3, 4 hindurchgehen, auf der Rückseite jedoch durch die andere Abdeckschicht abgedeckt sind. Die Druckknöpfe haben den besonderen Vorteil, dass sie eine Drehung der Befestigungsmittel nach dem Verbinden mit der Schiene ermöglichen. Auf diese Weise können die Befestigungsmittel optimal am geschienten Körperteil angebracht werden.

Ein zweites, sehr spezielles Ausführungsbeispiel in Form einer Fingerschiene (Metacarpalschiene) ist in Fig. 2A wiedergegeben. Die Schiene 9 besteht aus mehreren untereinander verbundenen Teilbereichen, nämlich einem sich nach vorne verjüngenden Fingerteil 10, an den sich nach hinten seitlich zwei zungenartige Stützteile 11 und 12 und in der Mitte ein Stützteller 13 anschliessen. Die Schiene 9 ist ebenso wie die Schiene 1 als Schichtenfolge aus unterer Abdeckschicht, gewelltem Metallblech und oberer Abdeckschicht aufgebaut. Die Wellung des Metallbleches ist durch gestrichelt eingezeichnete parallele Linien angedeutet. Im vorderen Fingerteil 10 verlaufen die Wellen 15 quer zur Schienenachse 45. Damit kann die Schiene in der o.g. Weise in Richtung der Pfeile 43 und 44 U-förmig um einen auf der Schiene 9 liegenden, zu schienenden Finger 28 (gestrichelt eingezeichnet) gebogen werden. Die Wellen 15 ermöglichen dabei insbesondere, dass der Fingerteil 10 nach dem Umbiegen in Richtung der Schienenachse 45 gekrümmt werden kann, so dass der geschiente Finger 28 in einer bevorzugten gekrümmten Position fixiert wird. Der Fingerteil 10 kann dabei - wie in Fig. 2A gezeigt - vor der Fingerkuppe enden; er kann aber auch länger sein, als der zu schienende Finger.

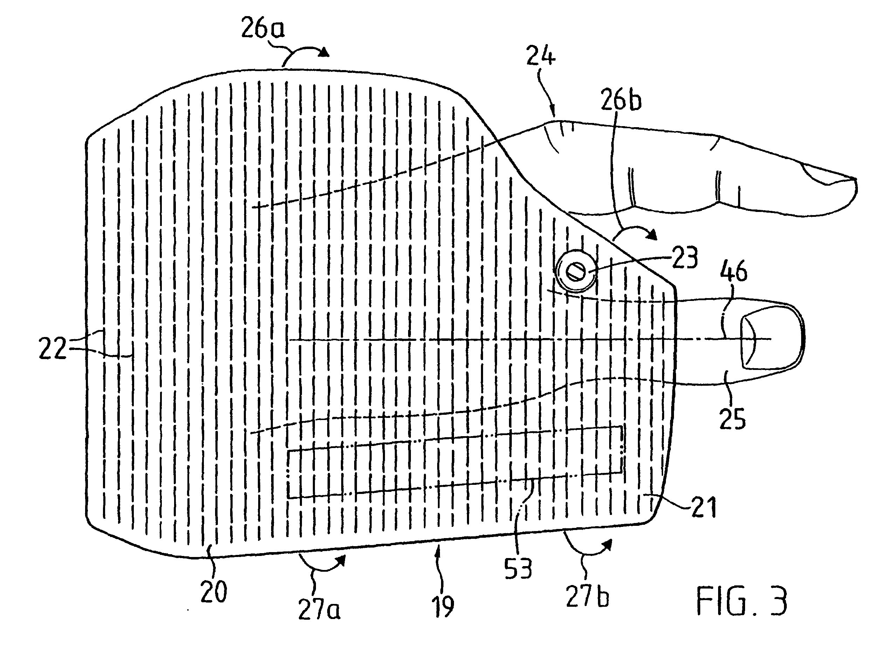

Im Bereich der Stützteile 11, 12 und des Stütztellers 13 sind die Wellen 16 im Metallblech parallel zur Schienenachse 45 orientiert. Damit wird erreicht, dass diese Teile, die den Fingerteil 10 an der Handfläche abstützen, eine erhöhte Stabilität aufweisen. Diese Teile können aber auch durch ein zusätzliches Blech verstärkt werden, so dass die Schiene in diesen Bereichen ein mehrfaches der üblichen Stärke erreicht. Zur Befestigung der Fingerschiene 9 an der Hand kann an der Schiene ein Riemen 17 gemäss Fig. 2B lösbar befestigt werden. Der Riemen 17, der aus einem länglichen Streifen 18 aus Schaumstoff oder Gewebe oder dgl. besteht, ist vorzugsweise am einen Ende mit einem Druckknopfteil 14b ausgerüstet. Der Riemen 17 kann - wenn er beispielsweise zusammen mit Schienen 19 bzw. 29 gemäss der Fig. 3 oder 4 verwendet wird - in diesem Bereich auch mit einer Einlage 47 aus einem versteifenden, plastisch verformbaren Material, z.B. aus ebenfalls gewelltem Al-Metallblech, versehen sein. Ebenso kann alternativ zwischen dem Riemen 17 und der Schiene 9 auch ein an beiden Enden mit Druckknopfteilen versehenes Zwischenstück aus relativ steifem, plastisch verformbaren Material vorgesehen werden, das mit dem einen Ende an der Schiene befestigt wird, und an dessen anderem Ende der Riemen 17 befestigt wird.

Das andere Druckknopfteil 14a als Gegenstück ist im Zentrum des Stütztellers 13 angeordnet. Wenn die Schiene 9 in der Handfläche positioniert und im Fingerteil 10 an den zu schienenden Finger 28 angeformt ist, wird der Riemen 17 (oder das o.g. Zwischenstück mit nachfolgendem Riemen) mit dem Druckknopfteil 14b in das Druckknopfteil 14a einrastend eingedrückt und der Riemen 17 mehrmals um die Hand geschlungen und z.B. mit Klebeband oder den üblichen Haken fixiert. Die Schiene 9 ist auf diese Weise sicher an der Hand fixiert.

Ein weiteres, in Fig. 3 dargestelltes Ausführungsbeispiel zeigt eine Schiene 19 mit spezieller (nicht-rechteckiger) Randkontur, die speziell als Daumenschiene Anwendung findet. Die Schiene 19 umfasst in diesem Fall einen breiten Handgelenkteil 20 und einen daran anschliessenden schmaleren und sich verjüngenden Daumenteil 21. Die Schiene kann aber auch kürzer und ohne Handgelenkteil ausgebildet sein und dient dann als reine Mittelhand-Daumenschiene, bei der das Handgelenk frei beweglich ist. Der innere Aufbau der Schiene 19 ist derselbe wie bei der Schiene 1 aus Fig. 1. Die (von aussen wegen der Abdeckschichten nicht sichtbaren) Wellen 22 im Metallblech sind durch die parallelen gestrichelten Linien angedeutet. Die Wellen 22 sind in diesem Fall durchgehend quer zur Schienenachse 46 angeordnet. Die Schiene 19 wird - wie in Fig. 3 gezeigt - seitlich auf die zu schienende Hand 24 aufgelegt, so dass der Daumenteil 21 mit der Schienenachse 46 parallel zum Daumen 25 liegt. Der Daumenteil 21 kann dabei - wie in Fig. 3 gezeigt - die Daumenkuppe freilassen; er kann aber auch den Daumen 25 vollständig der Länge nach überdecken. Die Schiene 19 wird dann in Richtung der Pfeile 26a, und 27a,b umgebogen und an die Hand 24 entsprechend angeformt. Der Daumen 25 ist dann im bezug auf die Hand 24 fixiert, so dass die Daumenwurzel ruhiggestellt ist. Durch eine Verlängerung der Schiene 19 zum Unterarm hin kann zusätzlich eine Fixierung (Ruhigstellung) des Handgelenks erreicht werden. Weiterhin kann es vorteilhaft sein, in bestimmten Bereichen der Schiene 19 (siehe auch Fig. 4A) eine Verstärkung 53, z.B. in Form eines zur Schienenachse 46 parallel verlaufenden Blechstreifens, einzubetten. Die Verstärkung sorgt dann nach dem Anpassen der Schiene 19 an den Daumen 25 für eine noch weiter verbesserte Fixierung. Zur Befestigung der Schiene 19 an der Hand sind im Bereich der Schiene 19, der zwischen Daumen 25 und Zeigefinger zu liegen kommt, wiederum Verbindungsmittel in Form eines eingelassenen Druckknopfteils 23 vorgesehen, in welche z.B. ein Riemen gemäss Fig. 2B (oder ein entsprechendes Zwischenstück) eingeknipst werden kann.

Ein weiterer Anwendungsfall ist die in Fig. 4 dargestellte Schienung des Unterarms bzw. Stütze des Handgelenks, wie sie beispielsweise im Falle einer Sehnenscheidenentzündung oder Operation notwendig sein kann. Die Schiene 29, die in diesem Fall neben demselben inneren Aufbau praktisch auch die allgemeine rechteckige Form der Schiene 1 gemäss Fig. 1 hat, wird in der gezeigten Weise auf den Unterarm 34 aufgelegt, dass sie über das Handgelenk 35 und die Fingerbasis hinausragt, die Finger selber aber überwiegend frei lässt. Die Schiene wird dann in Richtung der Pfeile 39, 40 um den Unterarm 34, das Handgelenk 35 und die Hand 33 U-förmig herumgebogen, wobei die quer zur Schienenachse 30 durchgehend verlaufenden Wellen 36 im Metallblech der Schiene für die Formbarkeit und Versteifung der Schiene sorgen. Zur Befestigung der Schiene 29 an der Hand 33 bzw. am Unterarm 34 kann auch hier als Verbindungsmittel ein Druckknopf 31 oder dgl. vorgesehen werden, in welchen ein Riemen gemäss Fig. 2B oder ein Unterstützungsteil 32 eingeknipst werden kann. Als Unterstützungsteil 32 kommt dabei insbesondere eine kleine Schiene in Betracht, die denselben Aufbau mit einem gewellten Metallblech hat, wie die Schiene 29, und durch den Zwickel zwischen Daumen 37 und Zeigefinger 38 hindurch hakenförmig umgebogen wird und so die Schiene 29 an der Hand 33 fixiert. Die Schiene 29 kann aber auch durch ein angeknöpftes und mehrfach um den Arm geschlungenenes Band gemäss Fig. 2B fixiert werden

Am freien Ende des Unterstützungsteils 32 kann dann zusätzlich ein Riemen gemäss Fig. 2B befestigt werden. Genausogut ist es aber auch denkbar, den Unterstützungsteil 32 als angeformten, seitlich herausstehenden Fortsatz der Schiene 29 auszubilden, so dass eine Befestigung der Schiene an der Hand 33 ohne Druckknopf und ohne zusätzliche, separate Befestigungsmittel erfolgen kann. Das gleiche Prinzip (des angeformten Fortsatzes) kann selbstverständlich auch im Zusammenhang mit der Daumenschiene gemäss Fig. 3 angewandt werden.

Ein weiteres Ausführungsbeispiel ist die in Fig. 4B dargestellte Schiene 48 in Form einer verkürzten Unterarm- bzw. Handgelenkstütze. Die Schiene 48 hat eine im wesentlichen rechteckige Form mit quer verlaufenden Wellen 50 und einem angeformten "Ohr" 51, in welchem ein Druckknopf 52 als Befestigungsmittel z.B. für ein Band gemäss Fig. 2B angeordnet ist. Die Schiene 48 wird in der dargestellten Weise (Pfeil) U-förmig um das Handgelenk der Hand 49 gebogen, so dass das Handgelenk fixiert ist, die Finger jedoch frei bewegt werden können. Die Schiene 48 kann dann mit einem angeknöpften Band gemäss Fig. 2B, welches zunächst zwischen zwischen Daumen und Zeigefinger hindurchgeführt und anschliessend mehrfach um das geschiente Handgelenk herumgewickelt wird, am Handgelenk fixiert werden. Um die Stabilität der Schiene 48 im Bereich des Handgelenks zu erhöhen kann zusätzlich eine Verstärkung 53 in Form eines eingelegten dickeren Blechstreifens (in Fig. 4A gestrichelt gezeichnet) vorgesehen werden, der Parallel zum Unterarm über das Handgelenk hinweg verläuft und in die Abdeckung mit eingeschweisst oder eingeklebt ist.

Insgesamt ergibt sich mit der Erfindung eine Schiene, welche leicht verformbar und an die zu schienenden Gliedmassen bzw. Körperteile anpassbar ist, trotz der hohen Steifigkeit (nach dem Verformen) eine überraschend hohe Verformbarkeit aufweist, und flexibel an die verschiedenen Anwendungsfälle anpassbar ist. Besonders günstig sind dabei Anwendungen als Arm-, Hand- oder Fingerschiene. Es sind aber ebenso auch Einsätze als Schiene im Bein- oder Fussbereich oder in anderen Körperregionen des Menschen (Hals, Wirbelsäule etc.), aber auch bei Tieren, denkbar.

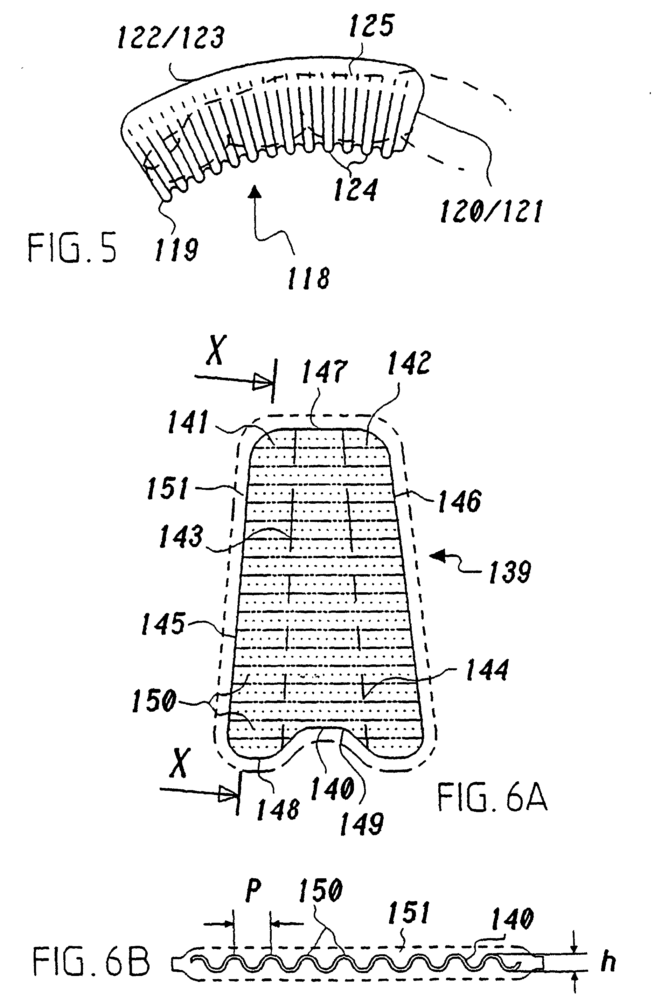

In Fig. 5 ist in Seitenansicht ein Ausführungsbeispiel einer Schiene nach der Erfindung in Form einer Fingerschiene wiedergegeben. Bei der Fingerschiene ist aus Gründen der Uebersichtlichkeit die an sich übliche Abdeckung mit einem elastischen Material, z.B. einer Schicht aus Polyäthylen- oder Polyurethanschaum, weggelassen. Die Fingerschiene 118 aus Fig. 5 besteht im wesentlichen aus einer der Länge eines Fingers 125 anpassbaren länglichen Platte 119 aus Aluminiumblech bzw. -folie, die in Längsrichtung wannenförmig gebogen wurde. Durch das Biegen entstanden zwei Seitenwände 120 und 121, die dem zu fixierenden Finger 125, der auf der Platte 119 in einem Mittelbereich aufliegt, seitlichen Halt zu geben und gleichzeitig der Fingerschiene eine Längssteifigkeit verleihen. Die zunächst in Längsrichtung geradlinige Schiene wird in der Regel anschliessend gekrümmt, um eine Fixierung des Fingers 125 in einer entspannten gekrümmten Position (sog. "physiologische Stellung") zu ermöglichen. Die Krümmung erfolgt so, dass im Bereich der Längskanten 122, 123 der Platte 119, die zugleich die Seitenwände 120, 121 nach oben begrenzen, eine Dehnung in Längsrichtung stattfindet. Um eine solche Dehnung zu ermöglichen, sind als Dehnungsmittel in der Platte 119 der Fingerschiene 118 Wellen 124 vorgesehen

Eine Fig. 5 entsprechende Fingerschiene 139 mit Wellen 150 für die Dehnung ist in Fig. 6A im ungebogenen Zustand in der Draufsicht wiedergegeben. Die Fingerschiene 139 aus Fig. 6A besteht im wesentlichen aus einer durch Längskanten 145, 146 und Querkanten 147, 148 begrenzten Platte 140, die von einer entsprechenden Abdeckung 151 umschlossen ist. Gedachte Biegelinien 143, 144 teilen Seitenabschnitte 141, 142 ab, die als Seitenwände der Schiene aus der Plattenebene herausgebogen werden sollen, um die Längsstabilität zu sichern und dem Finger oder den Fingern seitlichen Halt zu geben. Als Dehnungsmittel sind in diesem Ausführungsbeispiel Wellen 150 vorgesehen, die quer zur Längsrichtung der Platte 140 von einer Längskante 145 zur anderen Längskante 146 verlaufen und in Fig. 6A durch punktierte und strichpunktierte Linien angedeutet sind.

Die Platte 140 weist in diesem Beispiel nach vorne zu eine abnehmende Breite auf, die der zur Spitze hin schlanker werdenden Fingerform Rechnung trägt und bewirkt, dass die umgebogenen Seitenwände (120, 121 in Fig. 5) trotz der sich ändernden Fingerdicke eine annähernd konstante Höhe beibehalten können. Die Breite kann jedoch auch konstant gewählt werden, um z.B. eine vereinfachte Handhabung der Platte bei der Herstellung zu erreichen. Die Platte 140 ist an den Ecken vorzugsweise abgerundet, um die Verletzungsgefahr zu verringern. Die Platte 140 ist weiterhin durch eine Abdeckung 151 abgedeckt, die eine allseitig geschlossenen "Tasche" für die Platte 140 bildet und in Fig. 6 durch eine strichlierte Linie angedeutet ist. Eine solche Abdeckung in Form einer elastischen Schaumschicht ist z.B. in der Druckschrift US-A-4,676,233 ausführlich beschrieben, so dass hier auf eine nähere Erläuterung verzichtet werden kann. Es sei allerdings darauf hingewiesen, dass auch andere Materialien wie z.B. Kunststoffolien, Lacke oder textile Stoffe als Abdeckung geeignet sind.

Es versteht sich von selbst, dass die gedachten Biegelinien 143, 144 je nach der Form des zu fixierenden Fingers eine andere Lage und einen anderen Verlauf haben können oder auch gar nicht vorhanden sind, wenn das Hochbiegen der Seitenwände - wie bei der vorzugsweise wannenförmig gebogenen Schiene - stetig erfolgt. Um ein Drücken der Platte 140 am Fingeransatz zu verhindern, ist an der hinteren Querkante 148 der Platte 140 vorzugsweise eine Einbuchtung 149 vorgesehen.

Der Längsschnitt durch die Fingerschiene 139 entlang der Linie X-X in Fig. 6A ist in Fig. 6B wiedergegeben und zeigt, dass die Platte 140 als in Längsrichtung vorzugsweise fortlaufend gewellte Platte ausgebildet ist, wobei die Wellen 150 eine Periode P und eine Höhe h (zwischen Wellenberg und Wellental) haben. Da die Wellen 150 eine Versteifung der Platte 140 in Querrichtung bewirken und damit ein Umbiegen der Seitenabschnitte 141, 142 grundsätzlich erschweren, müssen Material und Dicke der Platte 140 sowie die Geometrie der Wellen geeignet gewählt werden, um die Fingerschiene für den Einsatz hinreichend leicht verformbar zu machen. Es hat sich dabei bewährt, wenn die Platte 140 aus Aluminium besteht, wenn die Platte 140 eine Dicke von kleiner gleich 0,5 mm, vorzugsweise kleiner gleich 0,2 mm aufweist, wenn die Wellen untereinander einen periodischen Abstand P von einigen Millimetern, vorzugsweise zwischen 0,5 mm und 5 mm, insbesondere etwa 2-3 mm, aufweisen, und wenn die Wellen eine Höhe h von wenigen Millimetern, vorzugsweise zwischen 0, 5 mm und 5 mm, vorzugsweise zwischen 1 mm und 3 mm, aufweisen. Besonders Aluminiumplatten bzw. -bleche mit einer Dicke von etwa 0,2 mm oder weniger gewährleisten eine gute Formbarkeit und gleichzeitige Stabilität der Fingerschiene.

Wenn eine Fingerschiene gemäss Fig. 6 mit gewellter Platte wannenförmig gebogen und anschliessend in Längsrichtung gekrümmt wird, ergibt sich der in Fig. 5 dargestellte Zustand. Die Fingerschiene 118 mit der gewellten Platte 119 umschliesst den Finger 125 mit den Seitenwänden 120 und 121. Durch die Krümmung der Schiene in Längsrichtung entsteht an den Längskanten 122 und 123 der Platte 119 eine starke Zugspannung, welche die Wellen 124 im Bereich der Längskanten nach innen zu abnehmend "glattzieht", so dass bei extremer Krümmung die Wellen an den Längskanten 122 , 123 vollständig verschwunden sind und sich die in Fig. 5 dargestellten geradlinigen Längskanten ausbilden. Mit dem "Glattziehen" der Wellen versteifen sich zugleich die Seitenwände 120, 121, so dass in diesem Fall die an sich gegenläufigen Forderungen nach Dehnbarkeit und Steifigkeit in optimaler Weise gleichzeitig erfüllt sind.

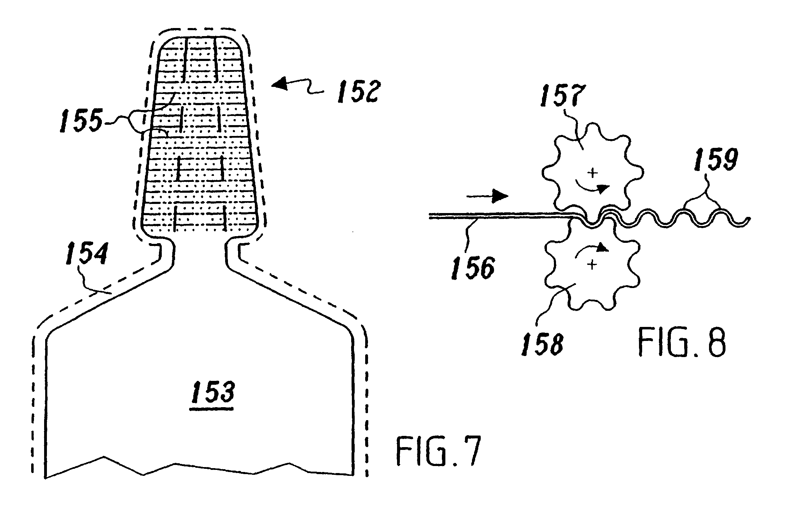

Die Fingerschienen der in Fig. 5 und 6 gezeigten Art werden zweckmässigerweise im ungebogenen Zustand bereitgehalten, so dass sie (ähnlich wie Wundpflaster oder dgl.) leicht verpackt und gelagert werden können. Grundsätzlich ist es denkbar für unterschiedliche Fingergrössen auch unterschiedliche Schienengrössen bereitzuhalten. Es hat sich jedoch herausgestellt, dass alle wesentlichen Anwendungsfälle mit einer Standardgrösse abgedeckt werden können, solange ein einzelner Finger geschient wird. Bei einer solchen Standardgrösse hat die Platte 119, 140 eine maximale Breite zwischen 4 cm und 10 cm, vorzugsweise etwa 6 cm, und eine maximale Länge zwischen 6 cm und 14 cm, vorzugsweise etwa 10 cm. Ueberstehende Bereiche der Schiene können zurückgebogen werden. Sind die Schienen für das gleichzeitige Schienen mehrerer Finger nebeneinander in einer Schiene vorgesehen, müssen die Platten in der Breite entsprechend grösser gewählt werden. Es ist auch denkbar, dass - wie in Fig. 7 gezeigt - die Fingerschiene 152 integraler Teil einer längeren und breiteren Armschiene 153 ist, und am vorderen Ende der Armschiene 153 angeformt ist. Hierdurch lässt sich bei Verletzungen an der Fingerbasis der Finger relativ zur Hand bzw. zum Arm fixieren, so dass eine ungestörte Heilung auch in diesem Fall möglich ist. Auch hier erweist sich eine Polsterung der Schienen 152 und 153 durch eine Abdeckung 154 als zweckmässig.

Die Fingerschiene mit gewellter Platte gemäss Fig. 5, 6 und 7 lässt sich auf unterschiedliche Weise herstellen. Besonders einfach ist eine Herstellung, bei der - wie aus Fig. 8 ersichtlich - eine ebene Platte 156 zur Erzeugung der Wellen 159 zwischen zwei in Eingriff stehenden Zahnrädern 157, 158 bzw. Zahnwalzen in Richtung des Pfeils hindurchgeführt wird. Die fertig gewellte Platte kann anschliessend mit der gewünschten Abdeckung versehen werden. Die Abdeckung kann aber durchaus auch vor der Wellung aufgebracht werden. Die Zähne der Zahnräder 157, 158 können unterschiedliche Formen haben. Es ist jedoch vorteilhaft, wenn die erzeugten Wellen 159 eine gleichmässig geschwungene Wellenlinie bilden, um Kerbeffekte in der Platte zu vermeiden, die beim Biegen zu einem Brechen der Platte führen könnten, und um das "Glattziehen" der Wellen beim Krümmen der Fingerschiene zu erleichtern.

Anstelle von Zahnrädern kann Platte aber auch auf verschiedene andere Arten gewellt werden. So ist es beispielsweise denkbar, die Platte zur Erzeugung der Wellen zwischen entsprechend gewellten Druckstempeln zu pressen, die entweder ebene Stempelflächen aufweisen und senkrecht gegeneinander gepresst werden, oder aber bogenförmige Stempelflächen aufweisen und aneinander abrollen. Die Druckstempel können dabei so ausgebildet sein, dass sie gleichzeitig eine Stanz- bzw. Schneidfunktion übernehmen und die Schiene mit der gewünschten Randkontur versehen.

### BEZEICHNUNGSLISTE

- 1: Schiene (allg. Schiene)
- 2: Metallblech
- 3: obere Abdeckschicht
- 4: untere Abdeckschicht
- 5: Randbereich
- 6: Schienenachse
- 7: Druckknopfteil
- 8: Oese
- 9: Schiene (Fingerschiene)
- 10: Fingerteil
- 11,12: Stützteil
- 13: Stützteller (Handfläche)
- 14a,b: Druckknopfteil
- 15: Welle
- 16: Welle
- 17: Riemen (Band)
- 18: Streifen (länglich)
- 19: Schiene (Daumenschiene)
- 20: Handgelenkteil
- 21: Daumenteil
- 22: Welle
- 23: Druckknopfteil
- 24: Hand
- 25: Daumen
- 26a,b: Pfeil (Biegerichtung)
- 27a,b: Pfeil (Biegerichtung)
- 28: Finger
- 29: Schiene (Handgelenkschiene)
- 30: Schienenachse
- 31,52: Druckknopf
- 32: Unterstützungsteil (Hilfsschiene)
- 33,49: Hand
- 34: Unterarm
- 35: Handgelenk
- 36,50: Welle
- 37: Daumen
- 38: Zeigefinger
- 39,40: Pfeil (Biegerichtung)
- 41: Bereich
- 42: Welle
- 43,44: Pfeil (Biegerichtung)
- 45,46: Schienenachse
- 47: Einlage
- 48: Schiene (Handgelenkstütze)
- 51: Ohr
- 53: Verstärkung
- 118: Fingerschiene
- 119: Platte
- 120,121: Seitenwand
- 122,123: Längskante
- 124: Welle
- 125: Finger
- 139: Fingerschiene
- 140: Platte
- 141,142: Seitenabschnitt
- 143,144: Biegelinie (gedacht)
- 145,146: Längskante
- 147: vordere Querkante
- 148: hintere Querkante
- 149: Einbuchtung
- 151,154: Abdeckung
- 150,155: Welle
- 152: Fingerschiene
- 153: Armschiene
- 156: Platte (ungewellt)
- 157,158: Zahnrad
- 159: Welle
- P: Periode (der Wellen)
- h: Höhe (der Wellen)

## Patentansprüche

1. Medizinische Schiene (1, 9, 19, 29, 48, 118, 139, 152) zum Fixieren bzw. Ruhigstellen von beweglichen Körperteilen, insbesondere Gliedmassen, eines Menschen oder eines Tieres entlang einer Schienenachse (6, 30), welche Schiene (1, 9, 19, 29, 48, 118, 139, 152) ein ohne zusätzliche Hilfsmittel mit der Hand plastisch verformbares Metallblech (2; 119, 140) umfasst, welches aus Aluminium besteht, eine Dicke von kleiner gleich 1 mm, vorzugsweise kleiner gleich 0,3 mm, aufweist, zumindest in Teilbereichen gewellt ausgebildet ist und beidseitig mit einer Abdeckung (3, 4; 151, 154) bedeckt ist, **dadurch gekennzeichnet, dass** die Berge und Täler der Wellen (15, 22, 36, 42, 50, 124, 150, 155, 159) im wesentlichen quer zur Schienenachse (6, 30) verlaufen, dass die Wellen (15, 22, 36, 42, 50; 124, 150, 155, 159) untereinander einen periodischen Abstand (P) von zwischen 1 mm und 8 mm, insbesondere etwa 3-5 mm, aufweisen, und dass die Wellen (15, 22, 36, 42, 50; 124, 150, 155, 159) eine Höhe zwischen den Wellenbergen und Wellentälern von zwischen 1 mm und 8 mm, insbesondere zwischen 2 mm und 5 mm, aufweisen.

2. Schiene nach Anspruch 1, **dadurch gekennzeichnet, dass** das Metallblech (2) über die gesamte Fläche gewellt ausgebildet ist, und die Wellen (15, 22, 36, 42, 50) quer zur Schienenachse (6, 30) zwischen gegenüberliegenden Rändern des Metallbleches (2) durchgehend ausgebildet sind.

3. Schiene nach einem der Ansprüche 1 oder 2, **dadurch gekennzeichnet, dass** die Abdeckung Abdeckschichten (3, 4) umfasst, welche aus einem Kunststoff, insbesondere aus einem Schaumstoff, vorzugsweise aus einem elastischen Polyäthylenoder Polyurethanschaum, bestehen.

4. Schiene nach Anspruch 3, **dadurch gekennzeichnet, dass** die Abdeckschichten (3, 4) über den Rand des Metallbleches (2) hinausragen und einen umlaufenden Randbereich (5) bilden, in welchem die beiden Abdeckschichten (3, 4) miteinander verbunden, vorzugsweise verklebt oder verschweisst, sind.

5. Schiene nach einem der Ansprüche 1 bis 4, **dadurch gekennzeichnet, dass** die Schiene (1, 29) eine im wesentlichen rechteckige Randkontur aufweist.

6. Schiene nach einem der Ansprüche 1 bis 4, **dadurch gekennzeichnet, dass** die Schiene (9, 19, 48) in ihrer Randkontur derart dem jeweiligen Einsatzbereich angepasst ist, dass an die Schiene zur Befestigung der Schiene an den zu schienenden Körperteilen bzw. Gliedmassen ein seitlich herausstehender Fortsatz angeformt ist.

7. Schiene nach einem der Ansprüche 1 bis 6, **dadurch gekennzeichnet, dass** an einer Stelle oder mehreren Stellen der Schiene (1, 9, 19, 29, 48) Mittel (7, 8, 14a, 23, 31, 52) zum lösbaren oder dauerhaften Verbinden der Schiene (1, 9, 19, 29, 48) mit zusätzlichen Befestigungsmitteln (17, 32) vorgesehen sind.

8. Schiene nach Anspruch 7, **dadurch gekennzeichnet, dass** die Verbindungsmittel einen in der Ebene der Schiene angebrachten Druckknopf (31, 52) bzw. Druckknopfteil (7, 14a, 23) umfassen.

9. Schiene nach Anspruch 8, **dadurch gekennzeichnet, dass** der Druckknopf (31, 52) bzw. Druckknopfteil (7, 14a, 23) auf der Rückseite von der dortigen Abdeckschicht (3 bzw. 4) abgedeckt ist.

10. Schiene nach Anspruch 7, **dadurch gekennzeichnet, dass** die Verbindungsmittel eine in der Ebene der Schiene angebrachte Oese (8) umfassen.

11. Schiene nach Anspruch 7, **dadurch gekennzeichnet, dass** die Verbindungsmittel einen Klettverbinder umfassen.

12. Schiene nach einem der Ansprüche 1 bis 11, **dadurch gekennzeichnet, dass** in vorbestimmten Bereichen der Schiene (48) eine zusätzliche Verstärkung (53), insbesondere in Form eines eingelegten Blechstreifens oder einer Blechplatte, vorgesehen ist.

13. Schiene nach Anspruch 1 in Form einer Fingerschiene (118, 139, 152) für die Fixierung eines einzelnen verletzten Fingers (125) oder mehrerer verletzter Finger, **dadurch gekennzeichnet, dass** das Metallblech eine längliche, in der Längsrichtung durch zwei Längskanten (122, 123; 145, 146) berandete Platte (119, 140) ist, dass die Platte (119, 140) von den Längskanten (122, 123; 145, 146) berandete Seitenabschnitte aufweist, welche aus der Plattenebene heraus gebogen werden können, um als Seitenwände (120, 121) dem in der Fingerschiene (118, 139, 152) gelagerten Finger (125) seitlich Halt zu geben und gleichzeitig einen Beitrag zur Längssteifigkeit der Fingerschiene zu leisten, und dass in den Seitenabschnitten (141, 142) eine Mehrzahl von in die Platte (119, 140, 156) eingebrachten Wellen (124, 150, 155, 159) vorgesehen sind, welche sich von den Längskanten (122, 123; 145, 146) aus quer in die Seitenabschnitte (141, 142) bzw. Seitenwände (120, 121) hinein erstrecken.

14. Fingerschiene nach Anspruch 13, **dadurch gekennzeichnet, dass** die Wellen (124, 150, 155, 159) als zwischen den beiden Längskanten (122, 123 bzw. 145, 146) durchgehende Wellen ausgebildet sind.

15. Fingerschiene nach Anspruch 14, **dadurch gekennzeichnet, dass** die Platte (119, 140) zumindest abschnittsweise als in Längsrichtung fortlaufend gewellte Platte ausgebildet ist.

16. Fingerschiene nach Anspruch 15, **dadurch gekennzeichnet, dass** die Platte (119, 140) eine Dicke von kleiner gleich 0,5 mm, vorzugsweise kleiner gleich 0,2 mm, aufweist, dass die Wellen (124, 150, 155, 159) untereinander einen periodischen Abstand (P) von zwischen 0,5 mm und 5 mm, insbesondere etwa 2-3 mm, aufweisen, und dass die Wellen (124, 150, 155, 159) eine Höhe (h) zwischen den Wellenbergen und Wellentälern von zwischen 0,5 mm und 5 mm, vorzugsweise zwischen 1 mm und 3 mm, aufweisen.

17. Fingerschiene nach einem der Ansprüche 13 bis 16, **dadurch gekennzeichnet, dass** die Platte (119, 140) der Fingerschiene (118, 139, 152) nach vorne zu eine abnehmende Breite aufweist.

18. Fingerschiene nach einem der Ansprüche 13 bis 16, **dadurch gekennzeichnet, dass** die Platte der Fingerschiene eine konstante Breite aufweist.

19. Fingerschiene nach einem der Ansprüche 17 oder 18, **dadurch gekennzeichnet, dass** die Platte (119, 140) eine maximale Breite zwischen 4 cm und 10 cm, vorzugsweise etwa 6 cm, und eine maximale Länge zwischen 6 cm und 14 cm, vorzugsweise etwa 10 cm, aufweist.

20. Fingerschiene nach einem der Ansprüche 13 bis 19, **dadurch gekennzeichnet, dass** die Platte (119, 140) hinten durch eine hintere Querkante (148) begrenzt ist, und im Bereich der hinteren Querkante (148) eine Einbuchtung (149) aufweist.

21. Fingerschiene nach einem der Ansprüche 13 bis 20, **dadurch gekennzeichnet, dass** die Abdeckung (151, 154) aus einem Schaumstoff, vorzugsweise aus einem elastischen Polyäthylenoder Polyurethanschaum, besteht.

22. Fingerschiene nach einem der Ansprüche 13 bis 21, **dadurch gekennzeichnet, dass** die Fingerschiene (152) Teil einer längeren und breiteren Armschiene (153) ist, und am vorderen Ende der Armschiene (153) angeformt ist.

23. Verfahren zur Herstellung einer Fingerschiene nach Anspruch 13, **dadurch gekennzeichnet, dass** eine ebene Platte (156) zur Erzeugung der Wellen (59) zwischen zwei in Eingriff stehenden Zahnrädern (157, 158) bzw. Zahnwalzen hindurchgeführt wird.

24. Verfahren zur Herstellung einer Fingerschiene nach Anspruch 13, **dadurch gekennzeichnet, dass** eine ebene Platte zur Erzeugung der Wellen zwischen zwei entsprechend gewellten Druckstempeln gepresst wird.

25. Verfahren nach Anspruch 24, **dadurch gekennzeichnet, dass** mit dem Pressen gleichzeitig die Randkontur der Fingerschiene gestanzt bzw. geschnitten wird.

## Claims

1. Medical splint (1, 9, 19, 29, 48, 118, 139, 152) for fixing and immobilizing movable body parts, in particular limbs, of a human or an animal along a splint axis (6, 30), said splint (1, 9, 19, 29, 48, 118, 139, 152) comprising a sheet metal (2; 119, 140) which can be plastically deformed by hand without additional aids, which is made of aluminium, has a thickness of less than or equal to 1 mm, preferably less than or equal to 0.3 mm, is corrugated at least in some areas, and is covered on both sides with a covering (3, 4; 151, 154), **characterized in that** the peaks and valleys of the corrugations (15, 22, 36, 42, 50, 124, 150, 155, 159) run substantially transversely with respect to the splint axis (6, 30), **in that** the corrugations (15, 22, 36, 42, 50; 124, 150, 155, 159) have a periodic spacing (P) from one another of between 1 mm and 8 mm, in particular of approximately 3-5 mm, and **in that** the corrugations (15, 22, 36, 42, 50; 124, 150, 155, 159) have a peak-to-valley height of between 1 mm and 8 mm, in particular of between 2 mm and 5 mm.

2. Splint according to Claim 1, **characterized in that** the sheet metal (2) is corrugated across the entire surface, and the corrugations (15, 22, 36, 42, 50) are designed extending transversely with respect to the splint axis (6, 30) between opposite edges of the sheet metal (2).

3. Splint according to either of Claims 1 and 2, **characterized in that** the covering comprises cover layers (3, 4) which are made of a synthetic, in particular of a foam, preferably of an elastic polyethylene foam or polyurethane foam.

4. Splint according to Claim 3, **characterized in that** the cover layers (3, 4) protrude beyond the edge of the sheet metal (2) and form a peripheral edge area (5) in which the two cover layers (3, 4) are connected to one another, preferably bonded or welded.

5. Splint according to one of Claims 1 to 4, **characterized in that** the splint (1, 29) has a substantially rectangular edge contour.

6. Splint according to one of Claims 1 to 4, **characterized in that** the edge contour of the splint (9, 19, 48) is adapted to the respective area of application such that a laterally protruding continuation is integrally formed on the splint for the purpose of securing the splint to the body parts or limbs to be splinted.

7. Splint according to one of Claims 1 to 6, **characterized in that** means (7, 8, 14a, 23, 31, 52) for releasably or permanently connecting the splint (1, 9, 19, 29, 48) to additional fastening means (17, 32) are provided at one or more points on the splint (1, 9, 19, 29, 48).

8. Splint according to Claim 7, **characterized in that** the connection means comprise a press-button (31, 52) or press-button part (7, 14a, 23) arranged in the plane of the splint.

9. Splint according to Claim 8, **characterized in that** the press-button (31, 52) or press-button part (7, 14a, 23) is covered on the reverse by the cover layer (3 or 4) present there.

10. Splint according to Claim 7, **characterized in that** the connecting means comprise an eyelet (8) arranged in the plane of the splint.

11. Splint according to Claim 7, **characterized in that** the connecting means comprise a velcro-type fastener.

12. Splint according to one of Claims 1 to 11, **characterized in that** an additional reinforcement (53), in particular in the form of an inserted strip or plate of sheet metal, is provided at certain areas of the splint (48).

13. Splint according to Claim 1, in the form of a finger splint (118, 139, 152) for fixing a single injured finger (125) or several injured fingers, **characterized in that** the sheet metal is an elongate plate (119, 140) bordered in the longitudinal direction by two longitudinal edges (122, 123; 145, 146), **in that** the plate (119, 140) has side sections which are bordered by the longitudinal edges (122, 123; 145, 146) and which can be bent out from the plane of the plate in order to form side walls (120, 121) providing lateral support for the finger (125) held in the finger splint (118, 139, 152) and at the same time contribute to the longitudinal stiffness of the finger splint, and **in that**, in the side sections (141, 142), several corrugations (124, 150, 155, 159) are provided set into the plate (119, 140, 156) and extending transversely from the longitudinal edges (122, 123; 145, 146) into the side sections (141, 142) or side walls (120, 121).

14. Finger splint according to Claim 13, **characterized in that** the corrugations (124, 150, 155, 159) are designed as continuous corrugations between the two longitudinal edges (122, 123 and 145, 146).

15. Finger splint according to Claim 14, **characterized in that** the plate (119, 140), at least in some sections, is designed as a plate that is corrugated continuously in the longitudinal direction.

16. Finger splint according to Claim 15, **characterized in that** the plate (119, 140) has a thickness of less than or equal to 0.5 mm, preferably less than or equal to 0.2 mm, **in that** the corrugations (124, 150, 155, 159) have a periodic spacing (P) from one another of between 0.5 mm and 5 mm, in particular of approximately 2-3 mm, and **in that** the corrugations (124, 150, 155, 159) have a peak-to-valley height (h) of between 0.5 mm and 5 mm, preferably of between 1 mm and 3 mm.

17. Finger splint according to one of Claims 13 to 16, **characterized in that** the plate (119, 140) of the finger splint (118, 139, 152) decreases in width towards the front.

18. Finger splint according to one of Claims 13 to 16, **characterized in that** the plate of the finger splint has a constant width.

19. Finger splint according to one of Claims 17 or 18, **characterized in that** the plate (119, 140) has a maximum width between 4 cm and 10 cm, preferably approximately 6 cm, and a maximum length between 6 cm and 14 cm, preferably approximately 10 cm.

20. Finger splint according to one of Claims 13 to 19, **characterized in that** the plate (119, 140) is bordered at the back by a rear transverse edge (148) and has an indentation (149) in the area of the rear transverse edge (148).

21. Finger splint according to one of Claims 13 to 20, **characterized in that** the covering (151, 154) is made of a foam material, preferably of an elastic polyethylene foam or polyurethane foam.

22. Finger splint according to one of Claims 13 to 21, **characterized in that** the finger splint (152) is part of a longer and wider arm splint (153) and is integrally formed on the front end of the arm splint (153).

23. Method for producing a finger splint according to Claim 13, **characterized in that**, in order to generate the corrugations (59), a flat plate (156) is passed between two meshing gearwheels (157, 158) or toothed rollers.

24. Method for producing a finger splint according to Claim 13, **characterized in that**, in order to generate the corrugations, a flat plate is pressed between two suitably corrugated pressure stamps.

25. Method according to Claim 24, **characterized in that** the edge contour of the finger splint is punched and cut simultaneously with the pressing.

## Revendications

1. Attelle médicale (1, 9, 19, 29, 48, 118, 139, 152) pour la fixation ou l'immobilisation de parties corporelles mobiles, en particulier des membres d'une personne ou d'un animal, le long d'un axe d'attelle (6, 30), laquelle attelle (1, 9, 19, 29, 48, 118, 139, 152) comprend une feuille métallique (2 ; 119, 140) pouvant être déformable plastiquement à la main sans auxiliaire supplémentaire, laquelle se compose d'aluminium, présente une épaisseur inférieure ou égale à 1 mm, de préférence inférieure à 0,3 mm, est réalisée avec une ondulation au moins dans des régions partielles et est recouverte des deux côtés d'un revêtement (3, 4 ; 151, 154), **caractérisée en ce que** les sommets et les vallées des ondulations (15, 22, 36, 42, 50, 124, 150, 155, 159) s'étendent essentiellement transversalement à l'axe de l'attelle (6, 30), **en ce que** les ondulations (15, 22, 36, 42, 50 ; 124, 150, 155, 159) présentent entre elles une distance périodique (P) comprise entre 1 mm et 8 mm, en particulier environ entre 3 et 5 mm, et **en ce que** les ondulations (15, 22, 36, 42, 50 ; 124, 150, 155, 159) présentent une hauteur entre les sommets des ondulations et les vallées des ondulations comprise entre 1 mm et 8 mm, en particulier entre 2 mm et 5 mm.

2. Attelle selon la revendication 1, **caractérisée en ce que** la feuille métallique (2) est réalisée avec une ondulation sur toute la surface, et les ondulations (15, 22, 36, 42, 50) sont réalisées de manière continue transversalement à l'axe de l'attelle (6, 30) entre des bords opposés de la feuille métallique (2).

3. Attelle selon l'une quelconque des revendications 1 ou 2, **caractérisée en ce que** le revêtement comprend des couches de revêtement (3, 4) qui se composent d'une matière plastique, en particulier d'une mousse, de préférence d'une mousse de polyéthylène ou de polyuréthanne élastique.

4. Attelle selon la revendication 3, **caractérisée en ce que** les couches de revêtement (3, 4) dépassent au-delà du bord de la feuille métallique (2) et forme une région de bord périphérique (5) dans laquelle les deux couches de revêtement (3, 4) sont connectées l'une à l'autre, de préférence par collage ou par soudage.

5. Attelle selon l'une quelconque des revendications 1 à 4, **caractérisée en ce que** l'attelle (1, 29) présente un contour essentiellement rectangulaire.

6. Attelle selon l'une quelconque des revendications 1 à 4, **caractérisée en ce que** l'attelle (9, 19, 48) est adaptée par son contour périphérique à la région d'utilisation respective de telle sorte qu'une avancée saillant latéralement soit formé sur l'attelle afin de fixer l'attelle aux membres ou aux parties du corps à atteler.

7. Attelle selon l'une quelconque des revendications 1 à 6, **caractérisée en ce que** des moyens (7, 8, 14a, 23, 31, 52) pour l'assemblage desserrable ou durable de l'attelle (1, 9, 19, 29, 48) à des moyens de fixation supplémentaires (17, 32) sont prévus en un endroit ou en plusieurs endroits de l'attelle (1, 9, 19, 29, 48).

8. Attelle selon la revendication 7, **caractérisée en ce que** les moyens d'assemblage comprennent un bouton pression (31, 52) ou une partie de bouton pression (7, 14a, 23) disposé(e) dans le plan de l'attelle.

9. Attelle selon la revendication 8, **caractérisée en ce que** le bouton pression (31, 52) ou la partie de bouton pression (7, 14a, 23) est recouvert(e) au dos par la couche de revêtement (3, respectivement 4) qui s'y trouve.

10. Attelle selon la revendication 7, **caractérisée en ce que** les moyens d'assemblage comprennent un oeillet (8) prévu dans le plan de l'attelle.

11. Attelle selon la revendication 7, **caractérisée en ce que** les moyens d'assemblage comprennent une attache par bandes agrippantes.

12. Attelle selon l'une quelconque des revendications 1 à 11, **caractérisée en ce que** l'on prévoit, dans des régions prédéterminées de l'attelle (48), un renforcement supplémentaire (53), en particulier sous la forme d'un ruban de feuille métallique posé ou d'une plaque en feuille métallique.

13. Attelle selon la revendication 1, sous la forme d'une attelle pour un doigt (118, 139, 152) pour la fixation d'un seul doigt blessé (125) ou de plusieurs doigts blessés, **caractérisée en ce que** la feuille métallique est une plaque longue (119, 140), bordée dans la direction longitudinale par deux arêtes longitudinales (122, 123 ; 145, 146), **en ce que** la plaque (119, 140) présente des portions latérales bordées par les arêtes longitudinales (122, 123 ; 145, 146), lesquelles peuvent être repliées hors du plan de la plaque, afin de fournir un support latéral sous forme de parois latérales (120, 121) pour le doigt (125) placé dans l'attelle pour doigt (118, 139, 152) et pour assurer simultanément une contribution à la rigidité longitudinale de l'attelle pour doigt, et **en ce que** l'on prévoit dans les portions latérales (141, 142) une pluralité d'ondulations (124, 150, 155, 159) prévues dans la plaque (119, 140, 156), lesquelles s'étendent depuis les arêtes longitudinales (122, 123 ; 145, 146) transversalement vers l'intérieur dans les portions latérales (141, 142) ou les parois latérales (120, 121).

14. Attelle pour doigt selon la revendication 13, **caractérisée en ce que** les ondulations (124, 150, 155, 159) sont réalisées sous la forme d'ondulations continues entre les deux arêtes longitudinales (122, 123, respectivement 145, 146) .

15. Attelle pour doigt selon la revendication 14, **caractérisée en ce que** la plaque (119, 140) est réalisée au moins partiellement en tant que plaque ondulée de manière continue dans la direction longitudinale.

16. Attelle pour doigt selon la revendication 15, **caractérisée en ce que** la plaque (119, 140) présente une épaisseur inférieure ou égale à 0,5 mm, de préférence inférieure ou égale à 0,2 mm **en ce que** les ondulations (124, 150, 155, 159) présentent entre elles une distance périodique (P) comprise entre 0,5 mm et 5 mm, en particulier environ entre 2 et 3 mm, et **en ce que** les ondulations (124, 150, 155, 159) présente une hauteur (h) entre les sommets des ondulations et les vallées des ondulations comprise entre 0,5 mm et 5 mm, de préférence entre 1 mm et 3 mm.

17. Attelle pour doigt selon l'une quelconque des revendications 13 à 16, **caractérisée en ce que** la plaque (119, 140) de l'attelle pour doigt (118, 139, 152) présente une largeur se réduisant vers l'avant.

18. Attelle pour doigt selon l'une quelconque des revendications 13 à 16, **caractérisée en ce que** la plaque de l'attelle pour doigt présente une largeur constante.

19. Attelle pour doigt selon l'une quelconque des revendications 17 ou 18, **caractérisée en ce que** la plaque (119, 140) présente une largeur maximale comprise entre 4 cm et 10 cm, de préférence d'environ 6 cm, et une longueur maximale comprise entre 6 cm et 14 cm, de préférence d'environ 10 cm.

20. Attelle pour doigt selon l'une quelconque des revendications 13 à 19, **caractérisée en ce que** la plaque (119, 140) est limitée derrière par une arête transversale arrière (148) et présente un renfoncement (149) dans la région de l'arête transversale arrière (148).

21. Attelle pour doigt selon l'une quelconque des revendications 13 à 20, **caractérisée en ce que** le revêtement (151, 154) se compose d'une mousse, de préférence d'une mousse élastique de polyéthylène ou de polyuréthanne.

22. Attelle pour doigt selon l'une quelconque des revendications 13 à 21, **caractérisée en ce que** l'attelle pour doigt (152) fait partie d'une attelle de bras (153) plus longue et plus large, et est formée à l'extrémité avant de l'attelle pour bras (153).

23. Procédé de fabrication d'une attelle pour doigt selon la revendication 13, **caractérisé en ce qu'**une plaque plane (156) est introduite entre deux roues dentées s'engrenant (157, 158) ou deux cylindres dentés s'engrenant afin de produire les ondulations (59).

24. Procédé de fabrication d'une attelle pour doigt selon la revendication 13, **caractérisé en ce qu'**une plaque plane est pressée entre deux matrices de pression ondulées correspondantes pour produire les ondulations.

25. Procédé selon la revendication 24, **caractérisé en ce que** le contour du bord de l'attelle pour doigt est estampé ou découpé simultanément au pressage.
